# EUROPEAN PATENT APPLICATION

(11) **EP 4 296 671 A1**
(43) Date of publication of application: **27.12.2023**
(21) Application number: 22756276.6
(22) Date of filing: 17.02.2022
(51) Int. Cl.: G01N 33/53

(54) **MARKER FOR LYMPHOCYTIC ADENOHYPOPHYSITIS AND RELATED DISEASES, AND USE OF SAID MARKER**

(30) Priority: 17.02.2021 JP 2021023614
(71) Applicant: Fujita Academy, Toyoake-shi, Aichi 470-1192 (JP); National University Corporation Tokai National Higher Education and Research System, Nagoya-shi, Aichi 464-8601 (JP)
(72) Inventor: SUGIMURA Yoshihisa, Toyoake-shi, Aichi 470-1192 (JP); SUZUKI Atsushi, Toyoake-shi, Aichi 470-1192 (JP); FUJISAWA Haruki, Toyoake-shi, Aichi 470-1192 (JP); IWATA Naoko, Toyoake-shi, Aichi 470-1192 (JP); WATANABE Takashi, Toyoake-shi, Aichi 470-1192 (JP); KAIBUCHI Kozo, Nagoya-shi, Aichi 464-8601 (JP); NISHIOKA Tomoki, Nagoya-shi, Aichi 464-8601 (JP)
(74) Representative: J A Kemp LLP
(86) International application number: PCT/JP2022/006450
(87) International publication number: WO 2022/176958

(57) **Abstract**

The purpose of the present disclosure is to provide a technique that can contribute to less invasive or more reliable diagnosis of lymphocytic anterior hypophysitis and its related diseases. For this purpose, the KCNMA1 (calcium-activated potassium channel subunit alpha-1) protein or an epitope thereof is used as a diagnostic agent for lymphocytic anterior hypophysitis.

## Description

### Technical Field

The present Description relates to a marker for lymphocytic adenohypophysitis and related diseases, and to the use of the marker.

### Background Art

Autoimmune hypothalamic hypophysitis is one of the impairments of pituitary function for which the etiology is unclear, and lymphocytic hypophysitis is a disease representative of autoimmune hypothalamic hypophysitis. Lymphocytic hypophysitis can be classified, according to the location of a lesion, into lymphocytic adenohypophysitis (LAH), lymphocytic infundibuloneurohypophysitis (LINH), and lymphocytic panhypophysitis (LPH). LAH is a disease in which the inflammatory lesion is localized to the anterior pituitary and the secretion of anterior pituitary hormones, such as adrenocorticotropic hormone (ACTH), is reduced or abolished. LINH is a disease in which inflammation is localized in the infundibulum or posterior pituitary and central diabetes insipidus is produced. LPH is a disease in which inflammation is produced throughout the pituitary and clinical characteristics of both LAH and LINH are produced.

A characteristic feature of lymphocytic hypophysitis, e.g., LAH, is that its clinical symptoms, e.g., headache and visual disorders, are similar to those due to pituitary tumors as well as pituitary lesions arising from systemic diseases. Thus, MRI image acquisition, pituitary tissue biopsy, steroid administration with follow-up observation, and so forth are carried out to obtain a definitive diagnosis of lymphocytic hypophysitis.

Among the lymphocytic hypophysitides, the present inventors have already reported that a particular autoantibody acts as a disease marker for LINH (Patent Literature 1). A marker that differentiates isolated adrenocorticotropic (ACTH) deficiency (isolated ACTH deficiency, IAD), which is a disease related to LAH, has also already been reported (Patent Literature 2).

### Citation List

Patent Literature 1: Japanese Patent No. 5924502
Patent Literature 2: Japanese Patent Application Laid-open No. 2016-206072

### Summary of Invention

However, the definitive diagnosis of whether LAH is present is still problematic. Even when an MRI image is acquired in order to distinguish LAH from a pituitary tumor, cases, in which a definitive diagnosis is difficult after discrimination from a tumor, are not infrequent because, e.g., a local thickening is observed in common. In addition, biopsy, which enables a definitive diagnosis of LAH, is very invasive, e.g., shaving of the skeleton of the head/face. The definitive diagnosis of LAH using follow-up observation after steroid administration is also problematic because this requires very careful observation.

Patent Literature 2 provides a marker for IAD. IAD is a disease in which, among the anterior pituitary hormones, only the secretion of ACTH is impaired, causing adrenal insufficiency, and its pathology resembles that of LAH. Patent Literature 2 additionally states the viewpoint that the IAD marker can also function as an LAH marker. Thus, the discrimination of LAH from IAD remains problematic.

The present Description provides a technology that can contribute to a less invasive or more reliable diagnosis of lymphocytic adenohypophysitis and its related diseases.

### Solution to Technical Problem

As a result of performing proteomics analysis on samples from human subjects suffering from LAH, the present inventors identified over 600 proteins and identified 50 proteins as autoantigen candidates. The same analysis was also performed on human subjects suffering from IAD, and 131 proteins were identified as autoantigen candidates. Upon carrying out detailed analyses of these candidate proteins, the present inventors were able to identify autoantibodies that function as markers associated with the possibility of LAH morbidity. With regard to the LAH-related disease IAD, autoantibodies that function as markers associated with the possibility of IAD morbidity were also identified. The present Description provides the following based on this knowledge.
[1] A diagnostic agent for autoimmune hypothalamic hypophysitis, the agent containing one or two or more proteins, or an epitope thereof, selected from the group consisting of KCNMA 1 protein, GIT2 protein, FRAT1 protein, SLC1A5 protein, INADL protein, DOC2B protein, GATA2 protein, ZP1 protein, and PTPN13 protein.
[2] A diagnostic agent for lymphocytic adenohypophysitis, the agent containing KCNMA1 (calcium-activated potassium channel subunit alpha-1) protein or an epitope thereof.
[3] The diagnostic agent according to [2] further containing GIT2 (ARF GTPase-activating protein GIT2) protein or an epitope thereof.
[4] The diagnostic agent according to [2] or [3] further containing one or two or more proteins, or an epitope thereof, selected from the group consisting of FRAT1 (proto-oncogene FRAT1) protein, SLC1A5 (neutral amino acid transporter B(0)) protein, INADL (InaD-like protein Double) protein, DOC2B (C2-like domain-containing protein beta) protein, GATA2 (endothelial transcription factor GATA-2) protein, ZP1 (zona pellucida sperm-binding protein 1) protein, and PTPN13 (tyrosine-protein phosphatase non-receptor type 13) protein.
[5] A diagnostic agent for differentiating lymphocytic adenohypophysitis and isolated ACTH deficiency, the agent containing KCNMA1 protein or an epitope thereof, and one or two or more proteins, or an epitope thereof, selected from the group consisting of FRAT1 protein, SLC1A5 protein, INADL protein, DOC2B protein, GATA2 protein, ZP1 protein, and PTPN13 protein.
[6] The diagnostic agent according to [5] containing at least KCNMA1 protein, FRAT1 protein, and SLC1A5 protein or their epitopes.
[7] The diagnostic agent according to [5] or [6] containing at least INADL protein or an epitope thereof.
[8] The diagnostic agent according to any of [5] to [7] containing ZP1 protein and/or PTPN13 protein or an epitope thereof.
[9] The diagnostic agent according to any of [5] to [8] containing DOC2B protein and/or GATA2 protein or an epitope thereof.
[10] A diagnostic agent for isolated ACTH deficiency, the agent containing KCNMA1 protein or an epitope thereof.
[11] The diagnostic agent according to [10] further containing one or two or more proteins, or an epitope thereof, selected from the group consisting of GIT2 protein, FRAT1 protein, SLC1A5 protein, INADL protein, DOC2B protein, GATA2 protein, ZP1 protein, and PTPN13 protein.
[12] A test method for lymphocytic adenohypophysitis, the method detecting, as an index, an anti-KCNMA1 antibody in a sample obtained from an individual.
[13] A test method for differentiating lymphocytic adenohypophysitis from isolated ACTH deficiency, the method detecting, in a sample obtained from an individual, anti-KCNMA1 antibody and one or two or more selections from the group consisting of anti-FRAT1 antibody, anti-SLC1A5 antibody, anti-INADL antibody, anti-DOC2B antibody, anti-GATA2 antibody, anti-ZP1 antibody, and anti-PTPN13 antibody.
[14] A test method for isolated ACTH deficiency, wherein an index is one or two or more selections from the group consisting of anti-GIT2 antibody, anti-FRAT1 antibody, anti-SLC1A5 antibody, anti-INADL antibody, anti-DOC2B antibody, anti-GATA2 antibody, anti-ZP1 antibody, and anti-PTPN13 antibody, and/or an index is anti-KCNMA1 antibody, in a sample obtained from an individual.
[15] A test method for autoantibody related to lymphocytic hypophysitis, wherein an index is one or two or more antibodies selected from the group consisting of anti-KCNMA1 antibody, anti-GIT2 antibody, anti-FRAT1 antibody, anti-SLC1A5 antibody, anti-INADL antibody, anti-DOC2B antibody, anti-GATA2 antibody, anti-ZP1 antibody, and anti-PTPN13 antibody, in a sample obtained from an individual.
[16] A test kit for lymphocytic hypophysitis, the kit including one or two or more proteins or epitopes that immunologically bind to one or two or more antibodies selected from the group consisting of anti-KCNMA1 antibody, anti-GIT2 antibody, anti-FRAT1 antibody, anti-SLC1A5 antibody, anti-INADL antibody, anti-DOC2B antibody, anti-GATA2 antibody, anti-ZP1 antibody, and anti-PTPN13 antibody.
[17] A device for evaluating lymphocytic hypophysitis, the device including, on a solid phase, one or two or more proteins or epitopes that immunologically bind to one or two or more antibodies selected from the group consisting of anti-KCNMA1 antibody, anti-GIT2 antibody, anti-FRAT1 antibody, anti-SLC1A5 antibody, anti-INADL antibody, anti-DOC2B antibody, anti-GATA2 antibody, anti-ZP1 antibody, and anti-PTPN13 antibody.

### Brief Description of Drawings

FIG. 1 is a diagram that shows the distribution of antigen protein candidates discovered in the LAH patient group and IAD patient group in Example 1;
FIG. 2 contains FIG. 2(A) to FIG. 2(I), which show the results of Western blotting of autoantibodies identified in the individual sera of the LAH patient group and the IAD patient group in Example 2; and
FIG. 3 is a diagram that shows the distribution of autoantibodies identified in each of the LAH patient group and IAD patient group in Example 2.

### Description of Embodiments

The disclosure in the present Description relates to a technology that can contribute to a less invasive or more reliable diagnosis of LAH, i.e., lymphocytic adenohypophysitis, and its related diseases. In an embodiment of the disclosure of the present Description, the possibility of LAH morbidity is evaluated using as an index anti-KCNMA1 antibody in a sample obtained from an individual. The presence of anti-KCNMA1 antibody in, for example, blood, is associated with the possibility of LAH morbidity. As a consequence, by using anti-KCNMA 1 antibody in a sample from an individual as an index and carrying out detection, e.g., of whether or not it is present, useful information can be provided for affirming or denying the possibility of LAH morbidity in the individual. This can conveniently contribute to a reliable diagnosis of LAH.

In another embodiment of the disclosure of the present Description, the possibility of LAH morbidity and the possibility of IAD morbidity are evaluated using as an index anti-KCNMA1 antibody and one or two or more selections from the group consisting of anti-FRAT1 antibody, anti-SLC1A5 antibody, anti-INADL antibody, anti-DOC2B antibody, anti-GATA2 antibody, anti-ZP1 antibody, and anti-PTPN13 antibody, in a sample obtained from an individual. Anti-KCNMA1 antibody is associated with the possibility of LAH morbidity, while the IAD autoantibody group, e.g., anti-FRAT1 antibody and so forth, is associated with the possibility of IAD morbidity. As a consequence, useful information can be provided for affirming or denying the possibility of LAH morbidity and the possibility of IAD morbidity in an individual by detecting, e.g., whether or not anti-KCNMA1 antibody and one or two or more selections from the IAD autoantibody group are present in a sample from the individual. This can contribute to a diagnosis that differentiates LAH and IAD, which have been difficult to differentiate.

In another embodiment of the disclosure of the present Description, the possibility of isolated ACTH deficiency morbidity is evaluated using as an index one or two or more selections from the group consisting of anti-GIT2 antibody, anti-FRAT1 antibody, anti-SLC1A5 antibody, anti-INADL antibody, anti-DOC2B antibody, anti-GATA2 antibody, anti-ZP1 antibody, and anti-PTPN13 antibody, and/or using as an index anti-KCNMA 1 antibody, in a sample obtained from an individual. The presence of autoantibodies from the IAD autoantibody group including anti-GIT2 antibody is associated with the possibility of IAD morbidity. In addition, the presence of anti-KCNMA1 antibody is associated with the possibility of LAH morbidity and its absence is associated with the possibility of IAD morbidity. Due to this, useful information for affirming or denying the possibility of IAD morbidity can be provided by detecting, e.g., whether or not the IAD autoantibody group including anti-GIT2 antibody is present and/or whether or not anti-KCNMA 1 antibody is present. This can conveniently contribute to a reliable diagnosis of IAD.

The technology disclosed in the present Description does not, by itself, diagnose LAH and/or IAD, but does provide useful information for diagnosis. These diseases can be diagnosed by combining the technology disclosed in the present Description with observations and/or tests based on medical knowledge related to the diagnosis of LAH and IAD.

In the present Description, the possibility of LAH morbidity refers to the possibility of suffering from LAH at the time point of sample collection from the individual, and does not refer to the possibility of being affected by LAH in the future and does not refer to a sign of illness. The same applies to the possibility of IAD morbidity.

Various embodiments disclosed in the present Description are disclosed in the following.

### < Test method for lymphocytic adenohypophysitis (LAH) >

The test method for LAH disclosed in the present Description (also referred to in the following simply as the LAH test method) includes using, as an index, the anti-KCNMA1 antibody in a sample obtained from an individual. The use of the anti-KCNMA 1 antibody in a sample as an index, i.e., the detection, e.g., of whether or not it is present, provides useful information for affirming or denying the possibility of LAH morbidity in an individual. This can contribute to the diagnosis of the possibility of LAH morbidity.

The individual is typically a human. In addition, the sample obtained from an individual is a sample collected and isolated from the individual and should enable the detection, e.g., of whether the autoantibody that is the index in the LAH test method is present or not. For example, the sample is collected noninvasively or with minimal invasiveness. Typical examples are blood, plasma, serum, cerebrospinal fluid, urine, lacrimal fluid, saliva, and so forth. In addition, the sample may be, for example, cells or tissue collected from, e.g., a human subject. Such cells or tissue may be, for example, all or a portion of the hypothalamus or pituitary of an individual, for example, the anterior pituitary, the posterior pituitary, the hypothalamic infundibulum, and so forth.

The anti-KCNMA1 antibody is an antibody against the KCNMA1 (calcium-activated potassium channel subunit alpha-1) protein. Anti-KCNMAl antibody is an autoantibody broadly present in common in LAH patients and is a useful marker for affirming the possibility of LAH morbidity. The KCNMA1 protein is calcium-activated potassium channel subunit α-1. Anti-KCNMA1 antibody is a voltage-gated potassium channel and is also known as the large conductance calcium-activated potassium channel, subfamily M, alpha member 1 (KCal .1) or BK channel alpha subunit. The amino acid sequence of this protein is given, for example, in SEQ ID NO: 1.

The KCNMA1 protein in the present Description is not limited to that specified by this amino acid sequence. As long as the same functionality as protein composed of the amino acid sequence given by SEQ ID NO: 1 is present, it may contain mutations, e.g., SNPs, and may be an isoform. Thus, also included here is protein having the same functionality as protein composed of the amino acid sequence given by SEQ ID NO: 1 while having an amino acid sequence having an identity with the amino acid sequence given by SEQ ID NO: 1 of, for example, at least 70%; or for example, at least 80%; or for example, least 90%; or for example, at least 95%; or for example, at least 96%; or for example, at least 97%; or for example, at least 98%; or for example, at least 99%; or for example, at least 99.5%; or for example, at least 99.6%; or for example, at least 99.8%; or for example, at least 99.9%. For the other proteins considered in the present Description, there is also similarly no limitation to the protein having the amino acid sequence specified by the sequence number.

The LAH test method may carry out evaluation using anti-GIT2 antibody as an index, either by itself or in combination with anti-KCNMA1 antibody. Anti-GIT2 antibody is also an autoantibody in common to LAH patients and is a useful marker for affirming the possibility of LAH morbidity. Considering that anti-GIT2 antibody is also an autoantibody in common also for IAD patients, there may also be cases in which the possibility of LAH morbidity cannot be confirmed by only the presence of anti-GIT2 antibody.

GIT2 (ARF GTPase-activating protein GIT2) protein is a protein in the GIT protein family. GIT protein is believed to interact with G protein-coupled receptor kinase and to have an ADP ribosylation factor (ARF) GTPase activation protein (GAP) activity. The amino acid sequence of this protein is given, for example, in SEQ ID NO: 2.

The LAH test method may be a method in which evaluation is carried out additionally using as an index one or two or more selections from the group consisting of anti-FRAT1 antibody, anti-SLC1A5 antibody, anti-INADL antibody, anti-DOC2B antibody, anti-GATA2 antibody, anti-ZP1 antibody, and anti-PTPN13 antibody. These autoantibodies are autoantibodies present in common in IAD patients and are useful markers for affirming the possibility of IAD morbidity. If the possibility of IAD morbidity can be affirmed or denied by carrying out an evaluation using as an index one or two or more selections from this IAD autoantibody group, that is, by detecting, e.g., whether or not these are present, useful information can then be provided for affirming or denying the possibility of LAH morbidity at a higher degree of accuracy.

Here, the FRAT1 (proto-oncogene FRAT1) protein is a cancer-associated protein belonging to the GSK-3-binding protein family and is believed to possibly function in tumor progression and lymphoma formation. The amino acid sequence of this protein is given, for example, in SEQ ID NO: 3.

The SLC1A5 (neutral amino acid transporter B(0)) protein is a sodium-dependent amino acid transporter having a broad substrate specificity and a preference for zwitterionic amino acids. This protein accepts all the neutral amino acids as substrates, including glutamine, asparagine, and branched and aromatic amino acids, and excludes methylated, anionic, and cationic amino acids. The amino acid sequence of this protein is given in SEQ ID NO: 4.

The INADL (InaD-like protein Double) protein is a protein having a plurality of PDZ domains. The PDZ domain mediates protein-to-protein interactions, and a protein having a plurality of PDZ domains is believed to be able to organize multimeric complexes at plasma membranes. The amino acid sequence of this protein is given in SEQ ID NO: 5.

The DOC2B (C2-like domain-containing protein beta) protein is a protein that contains two C2-like domains and that enhances Ca⁽²⁺⁾-dependent exocytosis in adipocytes, adrenal chromaffin cells, and pancreatic beta-cells, and in the central nervous system DOC2B is believed to contribute to the spontaneous release of neurotransmitters. The amino acid sequence of this protein is given in SEQ ID NO: 6.

The GATA2 (endothelial transcription factor GATA-2) protein is a member of the GATA family of zinc finger transcription factors, and this protein is believed to play an important role in regulating the transcription of genes involved in the development and proliferation of hematopoietic and endocrine cell lineages. The amino acid sequence of this protein is given in SEQ ID NO: 7.

The ZP1 (zona pellucida sperm-binding protein 1) protein is a protein that ensures the structural integrity of the zona pellucida. The zona pellucida is the extracellular matrix that surrounds oocytes and early embryos and is primarily composed of three or four glycoproteins that have various functions during fertilization and preimplantation development. The amino acid sequence of this protein is given in SEQ ID NO: 8.

The PTPN13 (tyrosine-protein phosphatase non-receptor type 13) protein is a member of the tyrosine phosphatase (PTP) family. PTPs are believed to be signaling molecules that regulate various cellular processes, e.g., cell growth, differentiation, mitotic cycle, and oncogenic transformation. The amino acid sequence of this protein is given in SEQ ID NO: 9.

A combination with one or two or more selections from the IAD autoantibody group comprising the preceding can be used in the test method for differentiating LAH and IAD that is described below.

The evaluation in the LAH test method is carried out using these autoantibodies in a sample as an index. Blood, plasma, and serum are preferred for the sample from the standpoint of low invasiveness and considering convenience in the case of use of immunological methods. In addition, using collected cells and/or tissue as the sample is favorable in terms of enabling the acquisition of information, e.g., on the distribution and localization of autoantibodies in the cells or tissue. In order to use, for example, the presence/absence and/or the content of autoantibodies in a sample as an index, a pre-treatment may be carried out as appropriate in order to prepare from the sample, for example, a protein extract containing a high protein concentration.

There are no particular limitations on the detection of the autoantibody used as the index, but immunological methods based on antigen-antibody reactions can be adopted in view of the fact that autoantibodies are the target of the evaluation. Protein that is an antigen that immunologically binds to these autoantibodies, or an epitope of the protein, can be used as appropriate in the immunological methods. Here, epitope is a portion of an antigen that retains the same immunological binding capability as the antigen, and can be acquired as appropriate by the individual skilled in the art.

There are no particular limitations on the immunological method, which can be exemplified by latex agglutination methods, fluorescent immunoassays (FIAs), enzyme immunoassays (EIAs), radioimmunoassays (RIAs), Western blot methods, and immunoprecipitation methods (IPs). FIA and EIA (including ELISA) are examples of preferred assays. When the sample is cells or tissue, for example, immunocytochemical methods, immunohistochemical methods, and flow cytometry can be used.

For example, when ELISA is used, an antigen-immobilized solid phase is prepared in which antigen protein, which is an antigen for the autoantibody to be detected, is immobilized on a solid phase, e.g., a microplate. For example, a dilution of serum collected from a patient is supplied to this antigen-immobilized solid phase and an antigen-antibody complex is produced by an antigen-antibody reaction. A secondary antibody, which binds to the autoantibody and which has been preliminarily provided with a labeling component, for example, anti-human IgG antibody provided with horseradish peroxidase (HRP), is supplied to bring about the formation of an autoantibody-secondary antibody complex. The autoantibody can be detected and quantitated by then supplying a prescribed substrate for the HRP labeling component and carrying out observation at a specific absorption wavelength of the reaction product produced by the HRPmediated enzymatic reaction.

Various evaluation devices suitable for the LAH test method can be used to carry out such an immunological method in the LAH test method. For example, use can be made of a solid phase (microplate, array, chip, etc.) provided by the immobilization thereon of, e.g., antibody prepared for a competitive assay and/or one or two or more antigens to be simultaneously evaluated. Use can also be made of a strip (a carrier in which a liquid can move due to capillary phenomena) provided by the immobilization thereon of, e.g., a secondary antibody that binds autoantibody that has bonded with antigen. An antigen-antibody complex may be captured and detected by carrying out immunochromatography using this strip. In addition, using a strip having a DNA probe immobilized thereon, immunochromatography may be used to capture and detect a complex of the autoantibody with antigen that is bonded to a DNA strand complementary to the probe. Examples of the evaluation device are an antigen-packed immunochromatography column and immobilization beads in which antigen is immobilized on identifiable beads. Such arrays, strips, columns, beads, and so forth can be prepared as appropriate by the individual skilled in the art using known technology.

Various reagents suitable for the LAH test method can be used for execution of immunological methods in the LAH test method. For example, the following, inter alia, may also be used as appropriate: labeled or unlabeled secondary antibody to the antigen/autoantibody complex, labeling reagent for labeling the autoantibody, labeled autoantibody, labeled antigen or epitope, DNA strand-labeled antigen or epitope, and chromogenic reagents. These various reagents can be prepared as appropriate by the individual skilled in the art using known technology.

There are no particular limitations in the LAH test method on the mode of evaluating the possibility of LAH morbidity using the autoantibody in a sample as an index, and various modes can be adopted. For example, the presence/absence of autoantibody can be used as an index. That is, the presence/absence of an autoantibody in the sample may be qualitatively detected. The qualitative detection of the presence of an autoantibody means the detection, in the method for detecting the collected autoantibody, of a concentration (content) that is equal to or greater than the detection limit, with the detection limit being established as appropriate in accordance with, for example, the reference value for the presence/absence of common autoantibodies, the goal of the test method, the type of autoantibody, and so forth. The detection of the absence of autoantibody refers to a concentration (content) that is less than the aforementioned detection limit in the method for detecting the collected autoantibody.

For example, the autoantibody concentration may be used as an index. In this case, the autoantibody concentration may be quantitatively or semiquantitatively detected using the strength of a signal that is based on the autoantibody concentration. A concentration index and/or a standard curve is prepared as appropriate.

For example, a comparison of the autoantibody concentration with a predetermined reference value may be used as an index. In this case, for example, whether the autoantibody concentration is equal to or greater than (or exceeds) or is less than (or equal to or less than) a predetermined reference value is detected. The reference value can be set as appropriate based on measured values for the autoantibody concentration in the blood in a healthy subject group and a patient group having a confirmed disease diagnosis, such as an LAH patient group.

### < Diagnostic agent for lymphocytic adenohypophysitis (LAH) >

The LAH diagnostic agent disclosed in the present Description can detect, for example, the presence/absence of an autoantibody in a sample using the LAH test method described above. This diagnostic agent can provide useful information with regard to affirming or denying the possibility of LAH morbidity in an individual. In addition, some modes of this diagnostic agent can provide useful information with regard to denying the possibility of LAH morbidity in an individual. A convenient and definitive diagnosis of LAH is thereby made possible. This diagnostic agent is formulated to contain, in correspondence to the type of autoantibody used as the index in the LAH test method, antigen protein for this autoantibody or an epitope of this antigen protein. As has already been described, the antigen protein or epitope thereof contained in this diagnostic agent may be labeled as appropriate, may be in an immobilized state on a solid phase, and may assume the form of a device. When this diagnostic agent contains two or more antigen proteins or epitopes thereof, these are provided as a combination (kit) for diagnosis.

This diagnostic agent can contain, for example, KCNMA1 protein or an epitope thereof. In addition, this diagnostic agent can contain GIT2 protein or an epitope thereof, either by itself or in addition to KCNMA1 protein or an epitope thereof. Moreover, for example, in addition to the preceding, this diagnostic agent can also contain one or two or more proteins, or an epitope thereof, selected from the group consisting of FRAT1 protein, SLC1A5 protein, INADL protein, DOC2B protein, GATA2 protein, ZP1 protein, and PTPN13 protein. Which protein or epitope thereof is to be incorporated is determined as appropriate in correspondence to the design and goal of the test.

### < Test method for differentiating lymphocytic adenohypophysitis (LAH) and isolated ACTH deficiency (IAD) >

The test method disclosed in the present Description for differentiating LAH and IAD comprises using, as an index, anti-KCNMA1 antibody and one or two or more selections from the group consisting of anti-FRAT1 antibody, anti-SLC1A5 antibody, anti-INADL antibody, anti-DOC2B antibody, anti-GATA2 antibody, anti-ZP1 antibody, and anti-PTPN13 antibody, in a sample obtained from an individual. Information useful with regard to affirming or denying the possibility of LAH morbidity for an individual and information useful with regard to affirming or denying the possibility of IAD morbidity are provided by using, as an index, anti-KCNMA1 antibody and antibody selected from the IAD autoantibody group, i.e., anti-FRAT1 antibody and so forth, in a sample, that is, by detecting, for example, the presence/absence of these.

Only anti-KCNMA1 antibody is used for the possibility of LAH morbidity in this test method. Anti-GIT2 antibody is preferably not used since it affirms the possibility of both LAH morbidity and IAD morbidity.

The autoantibody from the IAD autoantibody group, e.g., anti-FRAT1 antibody and so forth, used in this test method is established as appropriate in correspondence to, e.g., the design and/or goal of the test. For example, anti-FRAT1 antibody and/or anti-SLC1A5 antibody can be included. These anti-IAD antibodies are widely in common in lAD patients because they are strongly associated with the possibility of IAD morbidity. In addition, for example, anti-INADL antibody from the IAD autoantibody group can be included. In addition, for example, anti-ZP1 antibody and/or anti-PTPN13 antibody from the anti-IAD antibody group can be included. This is due to an association with the possibility of IAD morbidity. For example, anti-DOC2B antibody and/or anti-GATA2 antibody from the anti-IAD antibody group can also be included. This is because both of these are associated with the possibility of IAD morbidity.

The modes described for the LAH test method can be used for the autoantibody detection method and detection mode in this test method. For example, when, in this test method, the presence of anti-KCNMA1 antibody is detected and autoantibody from the IAD autoantibody group is not detected, this provides useful information with regard to affirming the possibility of LAH morbidity and useful information with regard to denying the possibility of IAD morbidity. In addition, for example, when autoantibody that is anti-KCNMA1 antibody is detected and autoantibody from the IAD autoantibody group is also detected, this provides useful information with regard to affirming the possibility of both LAH morbidity and IAD morbidity. In addition, for example, when autoantibody that is anti-KCNMA1 antibody is not detected and autoantibody from the IAD autoantibody group is detected, this provides useful information with regard to denying the possibility of LAH morbidity and useful information with regard to affirming the possibility of IAD morbidity. In addition, for example, when neither of these autoantibodies is detected, this provides useful information with regard to denying the possibility of both morbidities.

### < Diagnostic agent for differentiating lymphocytic adenohypophysitis (LAH) and isolated ACTH deficiency (IAD) >

The diagnostic agent disclosed in the present Description for differentiating LAH and IAD can contain KCNMA1 protein or an epitope thereof, plus one or two or more proteins, or an epitope thereof, selected from the group consisting of FRAT1 protein, SLC1A5 protein, INADL protein, DOC2B protein, GATA2 protein, ZP1 protein, and PTPN13 protein. This diagnostic agent can provide useful information with regard to affirming or denying the possibility of LAH morbidity and the possibility of lAD morbidity in an individual. This makes it possible to more conveniently and more reliably diagnose LAH and IAD with respect to each other. This diagnostic agent is formulated to contain, in correspondence to the types of autoantibodies used as indices in the test method for differentiating LAH and IAD, antigen protein or epitope thereof for the autoantibodies. This diagnostic agent uses only KCNMA1 protein or epitope thereof as an index for the possibility of LAH morbidity. In addition, with regard to the index for the possibility of IAD morbidity, this diagnostic agent is formulated to contain, in correspondence to the type of autoantibody used as an index for the possibility of IAD morbidity, antigen protein or epitope thereof for the autoantibody. The same modes already described for the LAH test method and diagnostic agent can also be adopted for the various configurations of this diagnostic agent.

### < Test method for isolated ACTH deficiency (IAD) >

The IAD test method disclosed in the present Description comprises using, as an index, one or two or more selections from the group consisting of anti-GIT2 antibody, anti-FRAT1 antibody, anti-SLC1A5 antibody, anti-INADL antibody, anti-DOC2B antibody, anti-GATA2 antibody, anti-ZP1 antibody, and anti-PTPN13 antibody, and/or using, as an index, anti-KCNMA1 antibody, in a sample obtained from an individual. Useful information with regard to affirming or denying the possibility of IAD morbidity is provided by detecting, e.g., the presence/absence in a sample of one or two or more autoantibodies selected from the aforementioned IAD autoantibody group. In addition, useful information with regard to affirming or denying the possibility of LAH morbidity is provided by detecting, e.g., the presence/absence of anti-KCNMA1 antibody. This can contribute to a diagnosis of affirmation or denial of the possibility of IAD morbidity in an individual.

As described in the preceding, the IAD test method also uses as an index autoantibody selected as appropriate from the aforementioned IAD autoantibody group. Anti-GIT2 antibody, anti-FRAT1 antibody, and anti-SLC1A5 antibody are autoantibodies widely in common in IAD patients and are strongly associated with the possibility of IAD morbidity. Because anti-GIT2 antibody is also associated with the possibility of LAH morbidity, there will also be instances in which this index is not used. In addition, there is also an IAD patient group for which anti-INADL antibody is in common and associated therewith; there is also an IAD patient group with which anti-DOC2B antibody and anti-GATA2 antibody are associated; and there is also an IAD patient group for which anti-ZP1 antibody and anti-PTPN13 antibody are in common and associated therewith.

The aforementioned antibodies can be used as indices, that is, for example, their presence/absence can be detected, in the IAD test method also using the same detection methods and detection modes as in the LAH test method.

### < Diagnostic agent for isolated ACTH deficiency (IAD) >

The IAD diagnostic agent disclosed in the present Description can contain one or two or more proteins, or an epitope thereof, selected from the group consisting of GIT2 protein, FRAT1 protein, SLC1A5 protein, INADL protein, DOC2B protein, GATA2 protein, ZP1 protein, and PTPN13 protein. This diagnostic agent can provide useful information with regard to affirming or denying for an individual. This diagnostic agent is formulated to contain, in correspondence to the type of autoantibody used as an index in the IAD test method, antigen protein or epitope thereof for the autoantibody. The same modes already described for the LAH test method and diagnostic agent can also be adopted for the various modes of this diagnostic agent.

### < Autoantibody test method, etc., for lymphocytic hypophysitis >

The autoantibody test method disclosed in the present Description for lymphocytic hypophysitis comprises using as an index one or two or more antibodies selected from the group consisting of anti-KCNMA1 antibody, anti-GIT2 antibody, anti-FRAT1 antibody, anti-SLC1A5 antibody, anti-INADL antibody, anti-DOC2B antibody, anti-GATA2 antibody, anti-ZP1 antibody, and anti-PTPN13 antibody, in a sample obtained from an individual. This test method can acquire an individual-specific, lymphocytic hypophysitis-related autoantibody pattern - e.g., the presence/absence, type, and amount of lymphocytic hypophysitis-related autoantibody - possessed by an individual. It can also contribute to a diagnosis, within the sphere of lymphocytic hypophysitides, of LAH morbidity or IAD morbidity. In addition, for example, a presentation pattern for autoantibody associated with LAH and associated with IAD can be acquired. This test method can also be carried out using the various modes described for the LAH test method.

The diagnostic agent disclosed in the present Description for lymphocytic hypophysitis is provided with one or two or more proteins, or an epitope thereof, that bind with one or two or more antibodies selected from the group consisting of anti-KCNMA1 antibody, anti-GIT2 antibody, anti-FRAT1 antibody, anti-SLC1A5 antibody, anti-INADL antibody, anti-DOC2B antibody, anti-GATA2 antibody, anti-ZP1 antibody, and anti-PTPN13 antibody. The modes for the various diagnostic agents described for the LAH test method and LAH diagnostic agent can also be adopted for this diagnostic agent.

The lymphocytic hypophysitis evaluation device disclosed in the present Description is provided with, on a solid phase, one or two or more proteins, or an epitope thereof, that bind with one or two or more antibodies selected from the group consisting of anti-KCNMA 1 antibody, anti-GIT2 antibody, anti-FRAT1 antibody, anti-SLC1A5 antibody, anti-INADL antibody, anti-DOC2B antibody, anti-GATA2 antibody, anti-ZP1 antibody, and anti-PTPN13 antibody. This device is a mode of the aforementioned diagnostic agent, and the device modes described for the LAH test method and LAH diagnostic agent can be adopted.

Based on the preceding, the nine species of antigen proteins or epitopes thereof disclosed in the present Description can be regarded as useful for the diagnosis of LAH, which is one of the lymphocytic hypophysitides, and for the diagnosis of IAD, which is an LAH-related disease, and is useful for autoimmune adenohypophysitis. The present Description accordingly provides a diagnostic agent for autoimmune hypothalamic hypophysitis, including lymphocytic hypophysitis, that contains one or two or more proteins, or an epitope thereof, selected from the group consisting of KCNMA1 protein, GIT2 protein, FRAT1 protein, SLCIA5 protein, INADL protein, DOC2B protein, GATA2 protein, ZP1 protein, and PTPN13 protein. In addition, the present Description also provides a diagnostic device for autoimmune hypothalamic hypophysitis, that is provided, on a solid phase, with one or two or more proteins, or an epitope thereof, that bind with one or two or more antibodies selected from the group consisting of anti-KCNMA1 antibody, anti-GIT2 antibody, anti-FRAT1 antibody, anti-SLC1A5 antibody, anti-INADL antibody, anti-DOC2B antibody, anti-GATA2 antibody, anti-ZP1 antibody, and anti-PTPN13 antibody.

### [Examples]

Examples are described in the following as specific examples in order to more specifically describe the disclosure of the present Description. The following examples are for a description of the disclosure of the present Description and do not limit the scope thereof.

### [Example 1]

The following were obtained: an anterior pituitary protein extract obtained by removal of the anterior pituitary from the rat, and IgG in the blood of patients who have been definitively diagnosed with LAH or IAD ([1] and [2] below). These were reacted using, as antigen for each patient, the anterior pituitary-derived protein to obtain antigen-autoantibody complexes; in addition, autoantibody candidate proteins were determined by separating and analyzing the antigens ([3] below). Based on the amino acid sequences of human proteins that corresponded to these proteins, full-length human proteins were synthesized by genetic recombination ([1] in Example 2). These synthetic protein candidates were reacted with blood collected from each patient group, and the antigens were separated as described above to determine the protein candidates for antigens for autoantibodies ([2] in Example 2). The details are given in the following.

### [1] Preparation of rat anterior pituitary protein extract

The anterior pituitary was removed from male SD rats. The removed anterior pituitary tissue was homogenized in lysis buffer (20 mM Tris/HCl, 120 mM NaCl, 1 mM EDTA, 1% SDS, PhosSTOP (registered trademark), protease inhibitor, pH 7.4), followed by ultrasound treatment, addition of DTT and reduction, and then centrifugation for 1 hour at 43,000 rpm; the supernatant was used as the anterior pituitary protein extract.

### [2] Isolation and purification of IgG

IgG was isolated and purified using an IgG Purification Kit (Cosmo Bio Co., Ltd.) from the blood of the following: three lymphocytic adenohypophysitis (LAH) patients (32-year old female, 15-year old female, and one sample from a patient from another hospital, for which gender and age were unknown) who had been definitively diagnosed by pituitary biopsy, three isolated ACTH insufficiency (IAD) patients (71-year old female, 69-year old female, and one sample from a patient from another hospital, for which gender and age were unknown), and two healthy individuals.

### [3] Immunoprecipitation, mass spectrometry, and data analysis

The purified IgG and rat anterior pituitary protein extract were mixed, Protein A beads were added, and inversion mixing was carried out for 16 hours at 4°C. After the immunoprecipitation, the antigen was eluted from the immunoprecipitate by washing and trypsin addition. The eluted antigen was reduced and alkylated, followed by trypsin digestion (in-solution digestion) and analysis with an LC-MS/MS system (Orbitrap Fusion). Autoantigen protein candidates were identified by analyzing the obtained MS data using Mascot software (Matrix Science Ltd.) based on, e.g., the SwissProt database. 50 autoantigen protein candidates were ultimate identified for the LAH patient group. 131 autoantigen protein candidates were identified for the IAD patient group.

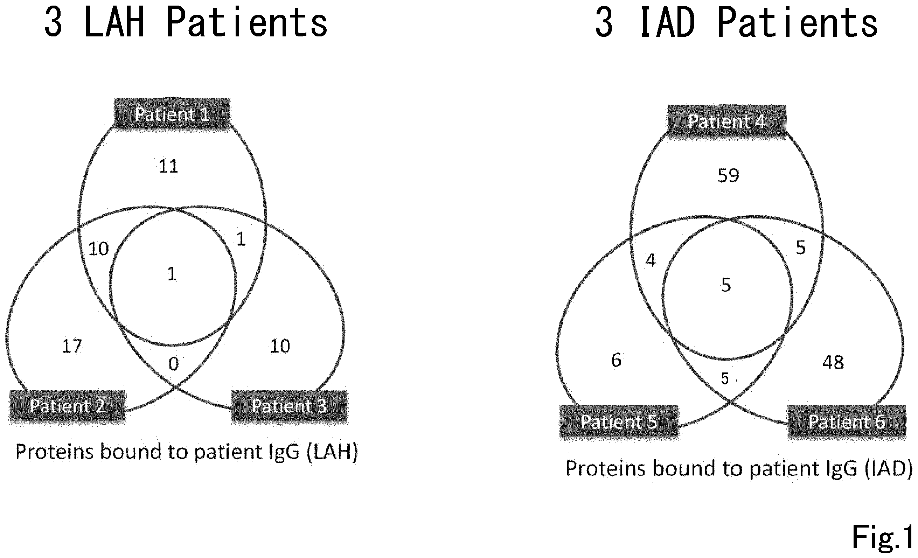

FIG. 1 shows the distribution of the autoantigen protein candidates in the LAH patient group and the IAD patient group. FIG. 1 contains the distribution of autoantigen proteins, from which, e.g., the proteins identified for the two healthy individuals functioning as the control group have been excluded. Of the autoantigen proteins shown in FIG. 1, those identified in at least two of the three patients in the LAH patient group and in at least two of the three patients in the IAD patient group were regarded as autoantigen candidates. As a result, 12 antigen proteins could be regarded as autoantigen candidates in the LAH patient group, and 19 antigen proteins could be regarded as autoantigen candidates in the IAD patient group.

### [Example 2]

### (Identification of autoantigens and diagnostic markers (autoantibodies) by Western blotting)

Then, for all of the autoantigen protein candidates present in common in the LAH patient group and the IAD patient group as obtained according to the results of the analysis shown in FIG. 1, recombinant full-length proteins were produced and the antigen-antibody reaction was analyzed by Western blotting using the sera from these patient groups and the healthy individuals as the primary antibody and using HRP-labeled anti-human IgG antibody as the secondary antibody. An autoantigen was taken to be antigen protein for which a band was observed in at least one of the three patients and for which a band was not observed in the two healthy individuals, and the corresponding antibody was taken to be a diagnostic marker.

### [1] Acquisition of recombinant full-length human protein

Using a known data base, base sequences were acquired and constructed that corresponded to the amino acid sequences of the autoantigen protein candidates obtained according to the results of the analysis shown in FIG. 1. In addition, in order to produce the full-length proteins, cDNAs having different regions were as necessary acquired from multiple sources.

For confirmation of the recognition by the sera of the LAH and IAD patients of the proteins KCNMA1, GIT2, Frat1; SLC1A5, INADL, DOC2B, GATA2, ZP1, and PTPN13, the amino acid sequences of the recombinant full-length human proteins corresponding to these are given, respectively, in SEQ ID NOs: 1 to 9. The full lengths of these proteins were acquired from the following resources.
(1) KCNMA1, synthetic DNA (Biotechnological Research Support Division, FASMAC Co., Ltd.)
(2) GIT2, Riken Gene Engineering Division, synthetic DNA (Integrated DNA Technologies, Inc.)
(3) Frat1, synthetic DNA (Integrated DNA Technologies, Inc.)
(4) SLC1A5, Riken Gene Engineering Division
(5) INADL, Riken Gene Engineering Division, K. K. DNAFORM, National Institute of Technology and Evaluation
(6) DOC2B, K. K. DNAFORM
(7) GATA2, Addgene
(8) ZP1, Riken Gene Engineering Division, synthetic DNA (Integrated DNA Technologies, Inc.)
(9) PTPN13, Harvard University

Except for the synthetic DNA, each open reading frame was amplified by PCR as appropriate. The synthetic DNA or PCR product was inserted into the mammalian expression vector pcDNA3.1-V5-His (Thermo Fisher Scientific Inc., Waltham, Massachusetts, USA) using Gibson assembly (NEB, Ipswich, Massachusetts, USA), and the sequences were read through using a Genetic Analyzer Sequencer (Thermo Fisher Scientific Inc.). Mutations were corrected as necessary.

HEK293FT cells cultured on a 12-well plate were transfected with each vector or a control blank vector according to the manufacturer's handling instructions using Lipofectamine 3000 reagent (Thermo Fisher Scientific Inc.). After 24 hours, the transfected HEK293FT cells were collected. The expression of each recombinant protein was confirmed by Western blotting using anti-V5 antibody (Invitrogen Corporation).

### [2] Detection of anti-autoantigen protein antibody in patient sera and presentation ratio for each antibody

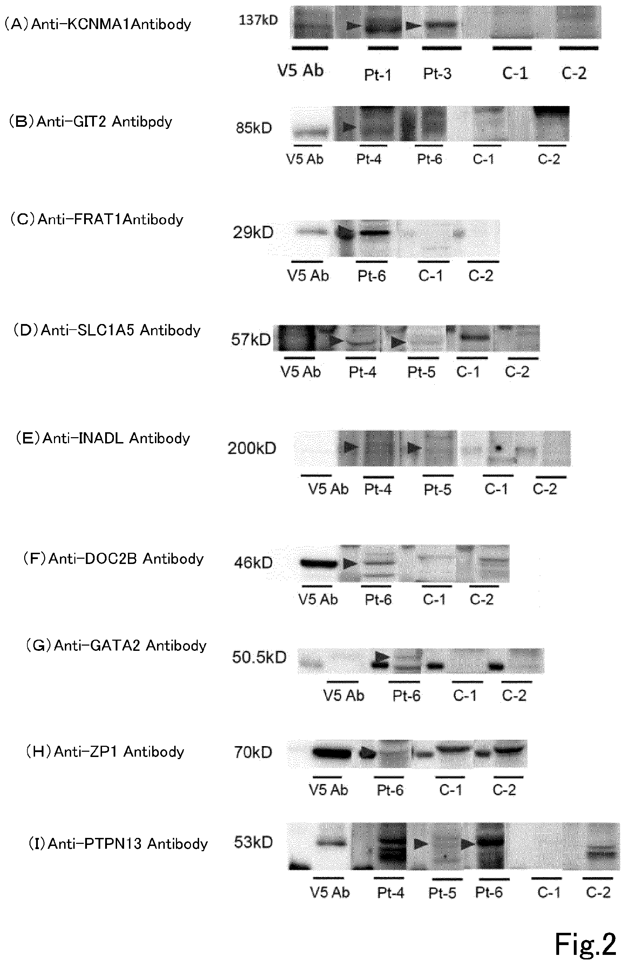

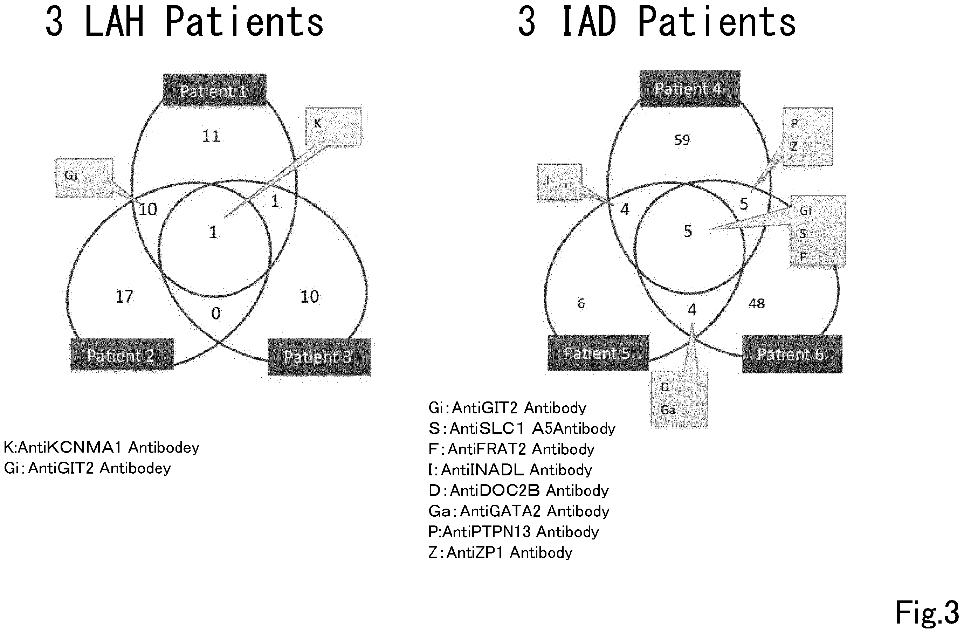

In order to evaluate the presence of antibody in the patient serum samples, a control lysate and each of the recombinant full-length human proteins produced by the HEK293FT cells in [1] above were subjected to electrophoresis on 7.5 to 10.00% polyacrylamide gel followed by transfer to a PVDF membrane; this was submitted to an immunoblotting procedure using each serum sample (1 : 50 dilution) or using anti-V5 antibody (1 : 1,000) as a positive control. The patient sera and sera from healthy individuals were used as the primary antibody; HRP-labeled anti-human IgG antibody was used as the secondary antibody; and the antigen-antibody reaction was analyzed by Western blotting. A protein for which a band was detected in a least one of the three LAH patients or at least one of the three IAD patients and for which a band was not detected in the two healthy individuals, was regarded as an autoantigen for that disease, and the detected antibody was regarded as a diagnostic marker (autoantibody) for that disease. Some of the results yielded by Western blotting are given in FIG. 2, while FIG. 3 gives the presentation results for the autoantibodies in each patient group based on the Western blotting results.

FIG. 2(A) shows the results of reaction with the serum from Patient 1 (Pt-1) and the serum from Patient 3 (Pt-3) by Western blotting using gel provided by the electrophoresis of recombinant full-length human protein that is an autoantigen candidate. As shown in FIG. 2(A), the patient sera reacted with KCNMA1 protein. The Pt-1 serum similarly reacted with GIT2 protein. The sera of healthy individuals C-1 and C-2 did not react with KCNMA1 protein or GIT2 protein. Based on this, it was found that the LAH patients presented anti-KCNMA 1 antibody and anti-GIT2 antibody.

FIG. 2(B) to FIG. 2(I) show the results of reaction with each of the sera from the IAD patients Pt-4, Pt-5, and Pt-6, by Western blotting using gel provided by the electrophoresis of recombinant full-length human protein that is an autoantigen candidate.

As shown in FIG. 2(B), the patient sera from Pt-4 and Pt-6 reacted with GIT2 protein. The sera from healthy individuals C-1 and C-2 did not react with GIT2 protein. Based on this, it was found that IAD patients presented anti-GIT2 antibody.

As shown in FIG. 2(C), the patient serum from Pt-6 reacted with FRAT1 protein. The sera from healthy individuals C-1 and C-2 did not react with FRAT1 protein. Based on this, it was found that IAD patients presented anti-FRAT1 antibody.

As shown in FIG. 2(D), the patient sera from Pt-4 and Pt-5 reacted with SLC1A5 protein. The sera from healthy individuals C-1 and C-2 did not react with SLC1A5 protein. Based on this, it was found that IAD patients presented anti-SLC1A5 antibody.

As shown in FIG. 2(E), the patient sera from Pt-4 and Pt-5 reacted with INADL protein. The sera from healthy individuals C-1 and C-2 did not react with INADL protein. Based on this, it was found that IAD patients presented anti-INADL antibody.

As shown in FIG. 2(F), the patient serum from Pt-6 reacted with DOC2B protein. The sera from healthy individuals C-1 and C-2 did not react with DOC2B protein. Based on this, it was found that IAD patients presented anti-DOC2B antibody.

As shown in FIG. 2(G), the patient serum from Pt-6 reacted with GATA2 protein. The sera from healthy individuals C-1 and C-2 did not react with GATA2 protein. Based on this, it was found that IAD patients presented anti-GATA2 antibody.

As shown in FIG. 2(H), the patient serum from Pt-6 reacted with ZP1 protein. The sera from healthy individuals C-1 and C-2 did not react with ZP1 protein. Based on this, it was found that IAD patients presented anti-ZP1 antibody.

As shown in FIG. 2(I), the patient sera from Pt-5 and Pt-6 reacted with PTPN13 protein. The sera from healthy individuals C-1 and C-2 did not react with PTPN13 protein. Based on this, it was found that IAD patients presented anti-PTPN13 antibody.

FIG. 3 is a diagram that shows, for the Venn diagrams of autoantigen protein candidates shown in FIG. 1, the distribution of autoantigens identified as autoantigens by Western blotting. As shown in FIG. 3, the following could be detected as autoantibodies (markers) according to the results of Western blotting: antibodies against nine proteins, i.e., KCNMA1, GIT2, Frat1, SLC1A5, INADL, DOC2B, GATA2, ZP1, and PTPN13, in at least two patients in the LAH patient group (patients 1 to 3) and the IAD patient group (patients 4 to 6). That is, the Western blotting results, supporting the proteomics results given in FIG. 1, showed that autoantibodies against these antigen proteins could be used as markers. It was also demonstrated that these antigen proteins could be used in a diagnostic agent as autoantigens for the particular disease.

Anti-KCNMA 1 antibody was discovered to be an autoantibody present in common in all the patients in the LAH patient group, as shown in FIG. 3. This autoantibody is strongly associated with the possibility of LAH morbidity. In addition, anti-GIT2 antibody was also discovered to be present in common in LAH patients. Both of these autoantibodies are novel.

Anti-GIT2 antibody, anti-FRAT1 antibody, and anti-SLC1A5 were also discovered to be autoantibodies present in common in all patients in the IAD patient group. Anti-INADL antibody was discovered to be present in common in two patients; anti-DOC2B antibody and anti-GATA2 antibody were also discovered to be present in common in two patients; and anti-ZP1 antibody and anti-PTPN13 antibody were also discovered to be present in common in two patients. These autoantibodies were also associated with the possibility of IAD morbidity. These autoantibodies are all novel.

Based on the preceding, nine autoantibodies, i.e., anti-KCNMA1 antibody, anti-GIT2 antibody, anti-FRAT1 antibody, anti-SLC1A5 antibody, anti-INADL antibody, anti-DOC2B antibody, anti-GATA2 antibody, anti-ZP1 antibody, and anti-PTPN13 antibody, were discovered to be autoantibodies associated with the possibility of LAH morbidity and IAD morbidity. These autoantibodies can provide useful information with regard to the possibility of LAH morbidity and/or IAD morbidity and can contribute to the diagnosis of LAH and/or IAD.

### [Example 3]

In this example, the anti-KCNMA1 antibody in the serum of patient Pt-1 was detected using KCNMA1 protein as the antigen protein.

### (1) Execution of ELISA procedure

### (1-1) Preparation of antigen-immobilized ELISA plate

An aqueous antigen solution (concentration = 2.5 µg/mL) was first prepared by dissolving the antigen in PBS. The aqueous antigen solution was dispensed onto an ELISA plate at 100 µL/well; standing at quiescence at 4°C was carried out overnight; the aqueous antigen solution was then discarded; and a blocking buffer was added at 100 µL/well and standing at quiescence overnight was carried out. The blocking buffer was then discarded, and storage with a desiccant at 4°C was carried out until use.

### (1-2) ELISA measurement

A patient serum dilution was first prepared by adding a reaction buffer to the patient serum. The patient serum dilution (1 : 101 dilution) was added at 100 µL/well to the prepared antigen-immobilized plate and a reaction was carried out for 2 hours at room temperature while shaking. The patient serum dilution was then discarded and the ELISA plate was washed using a wash buffer. Anti-human IgG (γ-chain)-HRP was then added as secondary antibody to the ELISA plate at 100 µL/well and shaking was performed for 1 hour at room temperature. The secondary antibody was discarded and the ELISA plate was washed with wash buffer. A reaction substrate solution was added at 4°C to the ELISA plate at 100 µL/well, and standing at quiescence was performed at room temperature for 15 minutes. A reaction stop solution was added at 100 µL/well to stop the reaction, and the 450 nm wavelength (620 nm secondary wavelength) was measured.

### (1-3) Results

Based on the results of measurement of the absorbance at wavelengths of 450 nm and 620 nm, the serum of patient Pt-1 reacted with KCNMA1 protein in the ELISA procedure and it could be confirmed that anti-KCNMA1 antibody was present in the patient serum. Using the other antigen proteins used as antigen proteins in Example 2, ELISA was run as above on the sera of the patients in whom autoantibodies against these antigen proteins had been detected, and their presence could be similarly confirmed. It was thus found that autoantibodies that could be detected in sera by Western blotting could also be similarly detected by ELISA.

## Claims

1. A diagnostic agent for autoimmune hypothalamic hypophysitis, the agent containing one or two or more proteins, or an epitope thereof, selected from the group consisting of KCNMA1 protein, GIT2 protein, FRAT1 protein, SLC1A5 protein, INADL protein, DOC2B protein, GATA2 protein, ZP1 protein, and PTPN13 protein.

2. A diagnostic agent for lymphocytic adenohypophysitis, the agent containing KCNMA1 (calcium-activated potassium channel subunit alpha-1) protein or an epitope thereof.

3. The diagnostic agent according to claim 2, further containing GIT2 (ARF GTPase-activating protein GIT2) protein or an epitope thereof.

4. The diagnostic agent according to claim 2 or 3, further containing one or two or more proteins, or an epitope thereof, selected from the group consisting of FRAT1 (proto-oncogene FRAT1) protein, SLC1AS (neutral amino acid transporter B(0)) protein, INADL (InaD-like protein Double) protein, DOC2B (C2-like domain-containing protein beta) protein, GATA2 (endothelial transcription factor GATA-2) protein, ZP1 (zona pellucida sperm-binding protein 1) protein, and PTPN13 (tyrosine-protein phosphatase non-receptor type 13) protein.

5. A diagnostic agent for differentiating lymphocytic adenohypophysitis and isolated ACTH deficiency, the agent containing KCNMA1 protein or an epitope thereof, and one or two or more proteins, or an epitope thereof, selected from the group consisting of FRAT1 protein, SLC1A5 protein, INADL protein, DOC2B protein, GATA2 protein, ZP1 protein, and PTPN13 protein.

6. The diagnostic agent according to claim 5, containing at least KCNMA 1 protein, FRAT1 protein, and SLC1A5 protein or their epitopes.

7. The diagnostic agent according to claim 5 or 6, containing at least INADL protein or an epitope thereof.

8. The diagnostic agent according to any of claims 5 to 7, containing ZP1 protein and/or PTPN13 protein or an epitope thereof.

9. The diagnostic agent according to any of claims 5 to 8, containing DOC2B protein and/or GATA2 protein or an epitope thereof.

10. A diagnostic agent for isolated ACTH deficiency, the agent containing KCNMA 1 protein or an epitope thereof.

11. The diagnostic agent according to claim 10, further containing one or two or more proteins, or an epitope thereof, selected from the group consisting of GIT2 protein, FRAT1 protein, SLC1A5 protein, INADL protein, DOC2B protein, GATA2 protein, ZP1 protein, and PTPN13 protein.

12. A test method for lymphocytic adenohypophysitis, the method detecting, as an index, an anti-KCNMA1 antibody in a sample obtained from an individual.

13. A test method for differentiating lymphocytic adenohypophysitis from isolated ACTH deficiency, the method detecting, in a sample obtained from an individual, anti-KCNMA1 antibody and one or two or more selections from the group consisting of anti-FRAT1 antibody, anti-SLC1A5 antibody, anti-INADL antibody, anti-DOC2B antibody, anti-GATA2 antibody, anti-ZP1 antibody, and anti-PTPN13 antibody.

14. A test method for isolated ACTH deficiency, wherein an index is one or two or more selections from the group consisting of anti-GIT2 antibody, anti-FRAT1 antibody, anti-SLC1A5 antibody, anti-INADL antibody, anti-DOC2B antibody, anti-GATA2 antibody, anti-ZP1 antibody, and anti-PTPN13 antibody, and/or an index is anti-KCNMA1 antibody, in a sample obtained from an individual.

15. A test method for autoantibody related to lymphocytic hypophysitis, wherein an index is one or two or more antibodies selected from the group consisting of anti-KCNMA1 antibody, anti-GIT2 antibody, anti-FRAT1 antibody, anti-SLC1A5 antibody, anti-INADL antibody, anti-DOC2B antibody, anti-GATA2 antibody, anti-ZP1 antibody, and anti-PTPN13 antibody, in a sample obtained from an individual.

16. A test kit for lymphocytic hypophysitis, the kit including one or two or more proteins or epitopes that immunologically bind to one or two or more antibodies selected from the group consisting of anti-KCNMA1 antibody, anti-GIT2 antibody, anti-FRAT1 antibody, anti-SLC1A5 antibody, anti-INADL antibody, anti-DOC2B antibody, anti-GATA2 antibody, anti-ZP1 antibody, and anti-PTPN13 antibody.

17. A device for evaluating lymphocytic hypophysitis, the device including, on a solid phase, one or two or more proteins or epitopes that immunologically bind to one or two or more antibodies selected from the group consisting of anti-KCNMA1 antibody, anti-GIT2 antibody, anti-FRAT1 antibody, anti-SLC1A5 antibody, anti-INADL antibody, anti-DOC2B antibody, anti-GATA2 antibody, anti-ZP1 antibody, and anti-PTPN13 antibody.
